# EUROPEAN PATENT SPECIFICATION

(11) **EP 2 510 823 B1**
(45) Date of publication and mention of the grant of the patent: **01.10.2014**
(21) Application number: 11161853.4
(22) Date of filing: 11.04.2011
(51) Int. Cl.: A44B 11/25, A61M 16/06

(54) **Assembly for connecting a headgear to a respiratory mask**
Anordnung zum Anschließen der Kopfkappe einer Beatmungsmaske
Ensemble de connexion de casque sur un masque respiratoire

(43) Date of publication of application: 17.10.2012
(73) Proprietor: Air Liquide Medical Systems, 92160 Antony (FR)
(72) Inventor: Alberici, Luca, 25128, Brescia (IT); Rivetti, Alberto, 25050, Rovato (BS) (IT); Lopez, Guillaume, 22560, Pleumeur Bodou (FR); Sandoni, Giuseppe, 25124, Brescia (IT); Masserdotti, Fulvio, 25075, Brescia (IT)
(74) Representative: Pittis, Olivier

(56) References cited:
- US-A1- 2004 045 551
- US-A1- 2006 225 740
- US-A1- 2010 258 133

## Description

The invention concerns an assembly comprising a connector element and a respiratory mask, such as a facial mask, for use in the treatment of respiratory conditions or diseases such as obstructive sleep apnea, said connector element being connected, on the one hand, to the mask body and, on the other hand, to the headgear of the mask.

Masks, such as facial or nasal masks, are commonly used for delivering non-invasive positive pressure ventilation (NPPV) or for nasal continuous positive airway pressure (N-CPAP) therapy in sleep disordered breathing (SDB) conditions such as obstructive sleep apnea (OSA). These masks typically deliver a flow of breathable gas for, or to assist in, patient respiration, especially during the night, i.e., when the patient is sleeping.

Such a mask assembly typically comprises a rigid or semi-rigid hollow shell, also called a mask body, defining a breathing chamber that receives at least a part of the patient's nose and/or mouth, and further comprising a soft face-contacting cushion that comes into contact with the patient's face, a forehead support that is optionally pivotable and a headgear for correctly positioning, maintaining and/or securing the mask on the head of a patient.

The shell of the mask or mask body is connected to a gas supply line which delivers a respiratory gas, such as air under pressure, into the breathing chamber of the shell through a gas opening or inlet orifice arranged in the wall of the mask body. The connection between the gas line and the mask body is usually achieved by means of a hollow connector comprising an internal passage for the gas. The connector can have an elbow-shape, i.e. can be of a curved shape or any other shape. A rotatable connector that rotates around the axis of the inlet orifice is known to be used to correctly position the gas line, which is typically a flexible conduit, with respect to the mask.

The soft face-contacting cushion often comprises two superimposed membranes, e.g., an inner membrane covered by an outer membrane, that ensures the air tightness all around the periphery of the cushion in contact with the user's face.

These types of respiratory masks are known from documents such as EP-A-1737524, EP-A-1841481, EP-A-956069 or EP-A-1118346.

More precisely, when the mask is used by the patient, the outer membrane comes into contact with the different regions of the user's face, i.e., the nasal bridge region, the chin region and the two lateral cheek regions that join the nasal bridge region to the chin region. As both membranes are made of a resilient flexible or soft material, the membranes are deformed under the traction forces exerted by the headgear of the mask in order to match the profile and forms of the user's face, thereby ensuring the air tightness of the mask.

However, the existing masks equipped with headgear are not totally satisfying. Indeed, the problem that exists is that connecting the straps of the headgear to the mask body is often not easy for the user, who because of the structure of the mask and the positioning necessary to put the mask into use is not able to see the site of connection while making the connection.

Further, US-A-2006/0225740 provides a connection device for connecting a headgear having straps, a mask and a system for supplying a gas flow to a patient. The connection device comprises a locking member configurated to lock the straps in a particular location; i.e. a male part with a connecting head having a bulbous or similar shape cooperating with a female part comprising a pair of parallel walls so that the bulbous head is maintained by the pair of walls when the male part is inserted into the female part.

Hence, the problem to be solved is to provide an improved connector element for respiratory masks, especially for facial masks, which allows an easy and quick coupling and release of the headgear to the mask body.

In other words, this problem is addressed through an assembly comprising a connector element and a mask body configured to allow a quick coupling and a quick release of the headgear. In addition, the assembly comprises simple structures.

The solution provided by the present invention is an assembly comprising a connector element and a respiratory mask having a mask body, and a coupling/releasing mechanism for coupling or uncoupling the connector element to the mask body. The coupling/releasing mechanism comprises a connecting pin having a semi-cylindrical head, the connecting pin cooperating with a wall structure that has an upper border in which an opening is arranged, the opening having an internal profile that matches the shape of the semi-cylindrical head of the connecting pin.

At least one of the wall structures is situated in a lodging arranged in the external wall of the mask body. Further, the peripheral border of each lodging is conformed and sized in order to have a profile that matches the peripheral contour of each connector body so that each connector body forms a roof that covers each of the lodgings. Preferably, the peripheral contour of each connector body has a "curved"-shape, such as a "(" or "C" shape.

Depending on the desired embodiment, the assembly of the present invention can comprise one or several of the following additional features:
- the connector element further comprises a connecting pin which has a semi-cylindrical head. Preferably, the connecting pin has an elongated semi-cylindrical head.
- the semi-cylindrical head of the connector element comprises an abutment at one end.
- the mask body includes a wall structure.
- the connector body has an inner face and an outer face, the inner face including the connecting pin and the outer face including a headgear-connecting structure.
- the connecting pin comprises a semi-cylindrical head and two lateral shoulders, with one of each of the two lateral shoulders arranged on each side of the semi-cylindrical head.
- each of the lateral shoulders arranged on each side of the semi-cylindrical head further comprises a blocking part for preventing or limiting the rotation of the semi-circular or semi-cylindrical head when inserted in the opening of the internal wall structure.
- each lodging forms a recess in the external wall of the mask body with each lodging further comprising a bottom, the mask body further comprising a breathing chamber and the bottom of each lodging projecting into the breathing chamber of the mask body.
- at least a wall structure, preferably each wall structure, is situated in, carried by or arranged on the bottom of the lodging, preferably of each lodging, with the wall structure projecting upward in relation to the positioning of the bottom.
- the wall structures and the mask body are molded into one piece, and are preferably made of a polymer material.
- the connector body, the connecting pin and the headgear-connecting structure are molded into one piece, and are preferably made of a polymer material.
- the polymer material utilized is chosen from the group comprising polycarbonate (PC), polypropylene (PP), acrylonitrile butadiene styrene (ABS), nylon and polystyrene (PS).
- the mask body further comprises a gas inlet orifice that is in fluid communication with the breathing chamber. Two lodgings, each having at least one wall structure, are arranged symmetrically with respect to the gas inlet orifice.
- at least one of the lodgings includes a bottom with at least one venting hole, preferably several venting holes, thereby ensuring a fluid communication between the lodging and the breathing chamber.

Furthermore, the mask of an assembly according to the present invention can further comprise one or more of the following additional features:
- the mask body further comprises a cushion and a headgear with one or more straps, at least one of said straps being attached to the headgear-connecting structure of the connector element.
- the mask body further comprises a forehead support, preferably a pivotable forehead support.
- the internal chamber comprises a peripheral border bearing the cushion.
- the cushion has a triangular or a saddle shape.
- the cushion comprises an inner membrane and an outer membrane.
- the cushion frame and the inner and outer membranes are molded into one piece and are preferably made of silicone.
- the outer membrane comprises a free curved edge projecting toward the interior of the cushion.
- the mask is a facial mask covering the nose and the mouth of the user.
- the mask further comprises a hollow gas-connector having an internal gas passage, such as an elbow-shaped connector, that is connected to a gas inlet orifice so as to be in fluid communication with the internal chamber of the mask.
- the hollow connector comprises an anti-asphyxia valve.

An embodiment of a connector element for a facial mask which is an embodiment of an assembly according to the present invention, as well as a facial mask connected to such a connector element are shown in the enclosed Figures, among which :
- Figure 1 represents an embodiment of an assembly comprising a facial mask and two connector elements according to the present invention when the mask is positioned on the patient's face and the connector elements are coupled to the mask body.
- Figure 2 is similar in nature to Figure 1 with the exception that the connector element is released.
- Figure 3 is a front view of the mask of Figure 1.
- Figure 4 is a lateral view of the mask of Figure 1.
- Figure 5 shows the outer face of the connector element.
- Figure 6 shows the inner face of the connector element.
- Figures 7 and 8 show a lodging with its internal wall structure receiving the connecting pin of the connector element.
- Figures 9, 10 and 12 show the inner structure of the breathing chamber of the mask body.

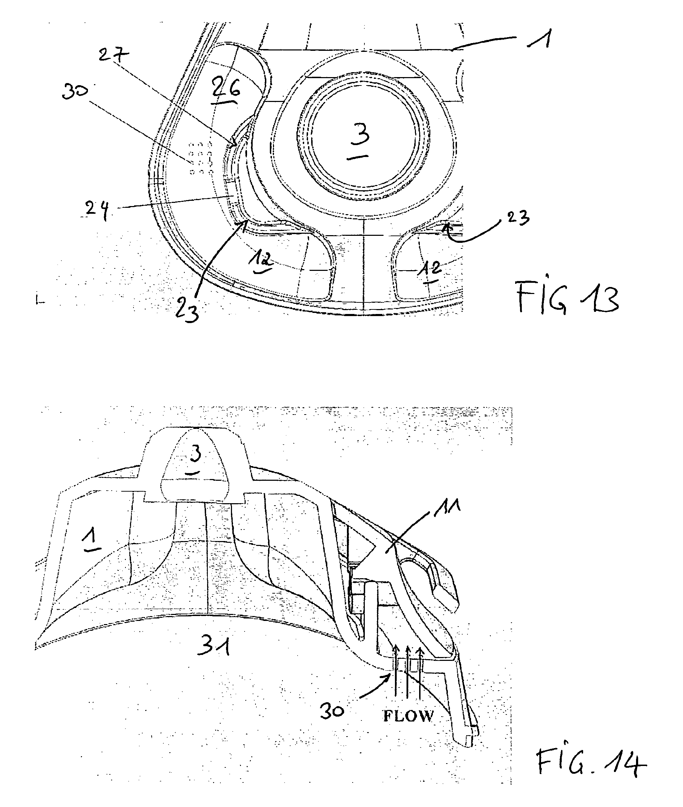

- Figures 11 and 14 provide a view in cross-section of the mask body of Figure 1 with and without the connector element coupled to the mask body.
- Figure 13 provides a front view of one of the lodgings of the mask of Figure 1.

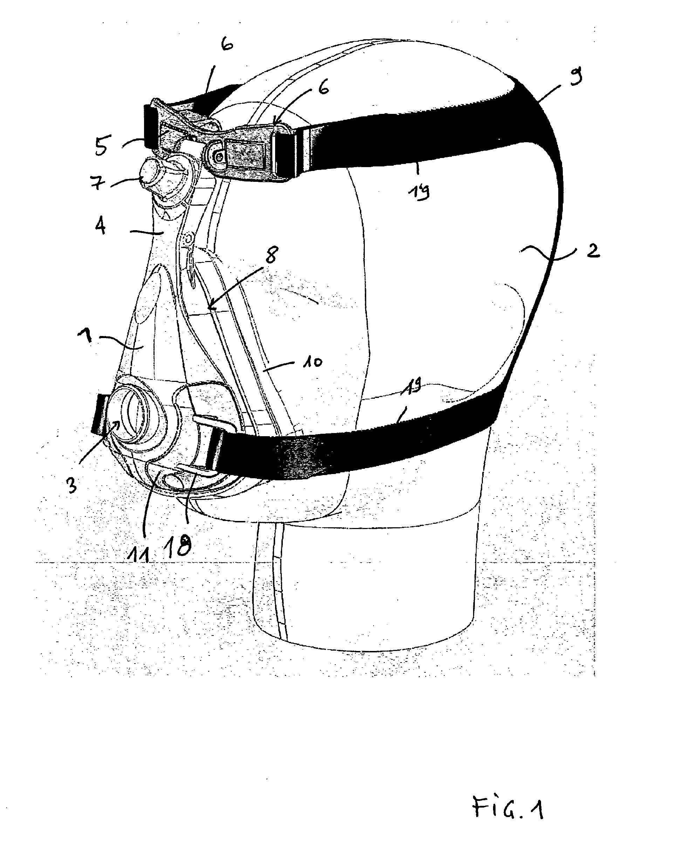

As illustrated in Figure 1, an embodiment of a respiratory facial mask of a mask/connector element assembly according to the present invention comprises a rigid or semi-rigid hollow shell or mask body 1 that defines an internal breathing chamber or volume wherein respiratory gas, such as air under pressure, is introduced via an inlet port 3 which is connected to a gas feeding line, such as a flexible gas conduit, by means of a tubular hollow connector. Preferably, the hollow connector has a general elbow-shape and comprises an internal passage for the gas. Advantageously, the hollow connector can rotate around the axis of the inlet port 3.

The gas inlet orifice 3 is arranged at the center of the mask body 1 and through the wall of the mask body 1 thereby allowing air under pressure to be introduced into the breathing chamber 31 that receives the nose and mouth of the patient 2.

The mask body 1 is preferably made of a polymer material, such as polycarbonate (PC), polypropylene (PP), acrylonitrile butadiene styrene (ABS), nylon or polystyrene (PS), and is configured so as to be able to receive at least a part of the patient's nose and mouth.

For breathing gas, the patient introduces his/her nose and mouth into the internal volume of the breathing chamber 31 of the mask body 1 and breathes the pressurized gas contained therein.

The mask body 1 has preferably a general triangular or quasi-triangular three-dimensional shape as demonstrated in Figure 1 for example.

The mask body 1 further comprises an expansion part 4 arranged on the top of the mask body 1 and projecting upwardly from the mask body 1, i.e. projecting away from the external surface of the mask body 1. The expansion part 4 and the mask body 1 are made of polymeric material that is molded into one piece so as to obtain an expansion part 4 that is integral with the rest of the mask body 1.

Further, as represented in Figure 1, a forehead support 5 is connected to the mask body 1 by means of a pivotable holding arm fixed to or carried by either the mask body 1 or the expansion part 4, preferably by the expansion part 4. The forehead support 5 can comprise one or more pillows 6 of soft and comfortable material that come into contact with the patient's forehead. An acting piece (not visible in Figure 1) is arranged in a traversing orifice of the expansion part 4 and is mobile, preferably in translation, in said traversing orifice so as to cooperate with the holding arm for pivoting said holding arm, when said acting piece moves in the traversing orifice of the expansion part 4. Further, the forehead support 5 can also pivot with respect to the holding arm 4. Actually, the back or forward motion of the acting piece in the traversing orifice is obtained by manual rotation by the user of a rotating knob 7 cooperating with the acting piece when said rotating knob 7 is manually operated, i.e. turned clockwise or counterclockwise, by the user. The maximum course of the knob 6 is about 360° or less.

Furthermore, in order to ensure a tight positioning of the mask on the patient's face and to increase the comfort for the patient 2, the peripheral border or edge 8 of the mask body 1 comprises a cushion 10 made of soft, resilient, elastomeric material that comes into contact with the patient's face. Said cushion 10 has a central aperture for receiving the patient's nose and mouth. The central aperture of the cushion 10 is positioned with regard to the internal chamber of the mask body 1, i.e. they are facing each other. The cushion 10 arranged on the peripheral border 8 of the internal chamber of the mask body 1 comprises two superimposed flexible membranes ensuring an air tightness to minimize air leaks. Such types of cushions 10 and mask body 1 structures are well-known in the art and taught by many documents, such as EP-A-1334742, EP-A-264772, EP-A-956069, EP-A-874667, US 2,931,356 or EP-A-1479406. More precisely, the border 8 and the cushion 10 have a general triangular or saddle-shape structure so as to match the contours of the nasal region including the upper nasal bridge region, the cheek regions on both sides of the nose, and the lower chin region of the patient 2.

The shell or mask body 1 is fluidly linked to a gas supply line, such as a flexible hose or conduit that conveys respiratory gas to the mask, by means of a tubular hollow connector which delivers a respiratory gas, such as air under pressure, into the breathing chamber of the shell 1 through the gas inlet 3 arranged in the mask body 1, as already mentioned above.

Gas under pressure, such as air, can be produced and delivered in a BPAP-type (bi-level positive airway pressure) or CPAP-type (continuous positive airway pressure) apparatus.

Further, a headgear 9 comprising straps 19 can be connected or disconnected to the mask body 1, by means of one or several connector elements 11 thereby maintaining the mask in a desired position on the patient's face during the use of the mask, thus allowing for an efficient treatment of sleep disorders, such as OSA or similar disorders.

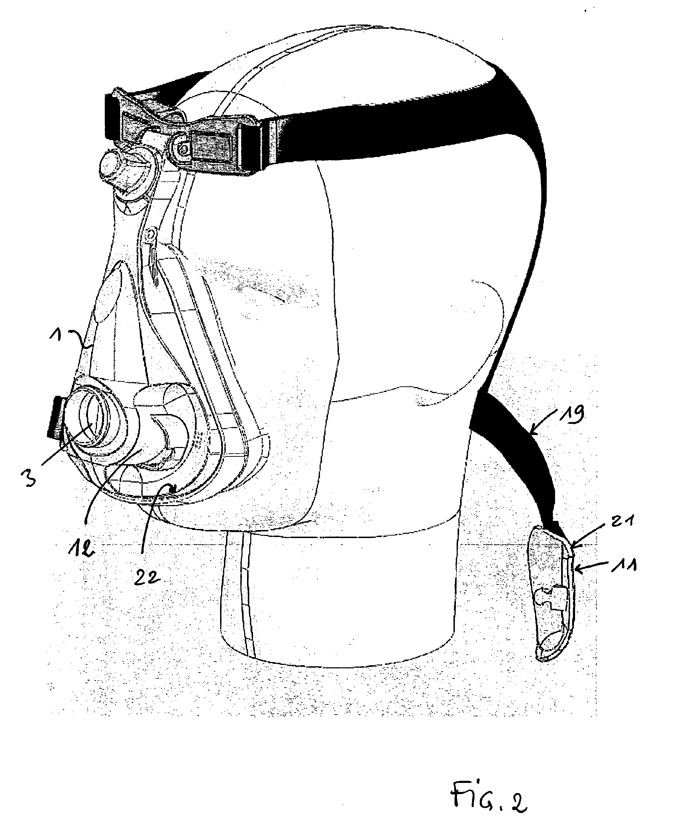

Preferably, the headgear 9 is coupled to the mask body 1 by means of two connector elements 11 as demonstrated in Figures 1 and 2.

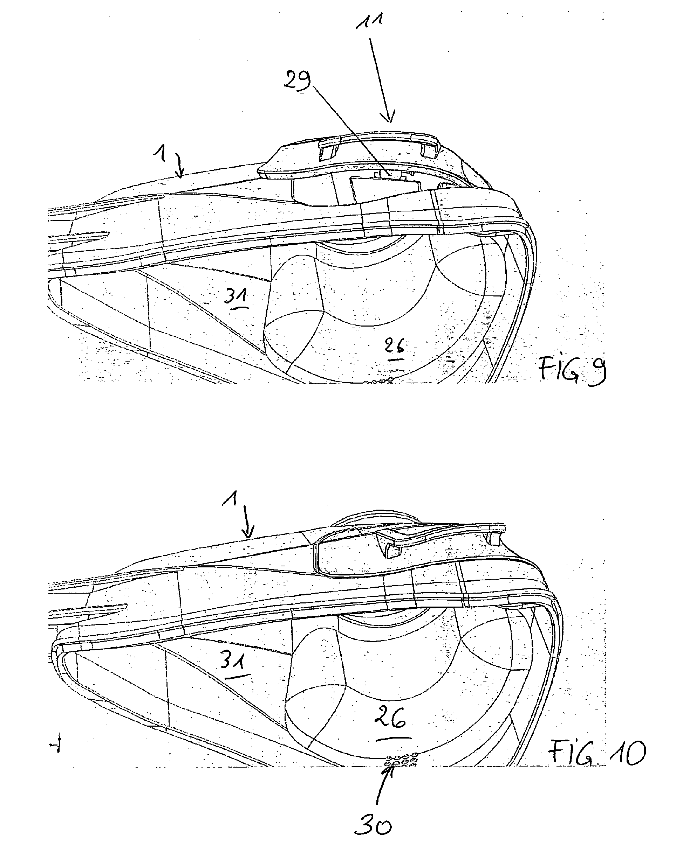

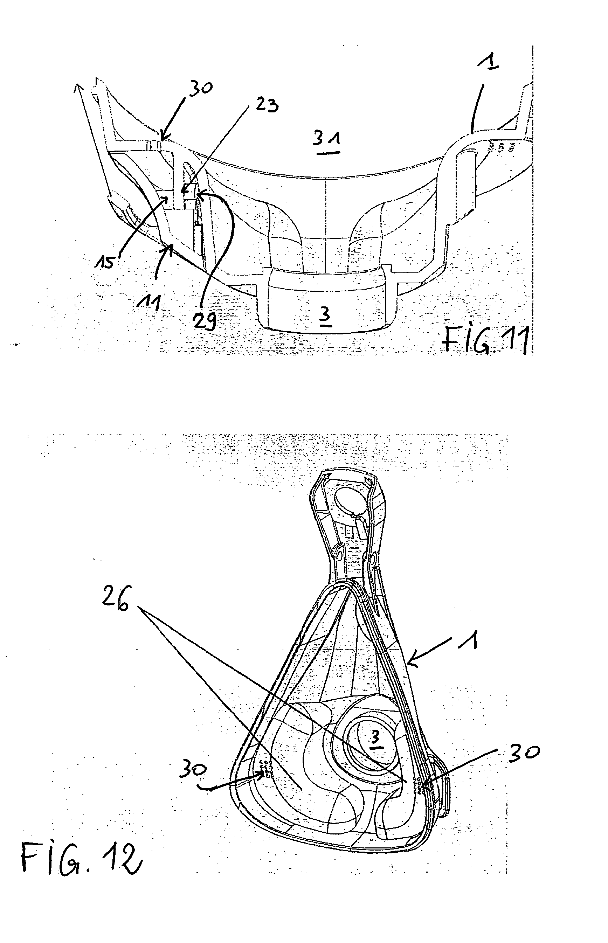

Each connector element 11 cooperates with a lodging 12 arranged in the wall of the mask body 1. More precisely, each lodging 12 is in the form of a recess that is made or formed into the external wall of the mask body 1 as shown in Figures 2, 6 and 8. The lodgings 12 form a kind of chamber that projects toward the interior (the inner volume) of the breathing chamber 31 as shown in Figures 9, 10 and 12.

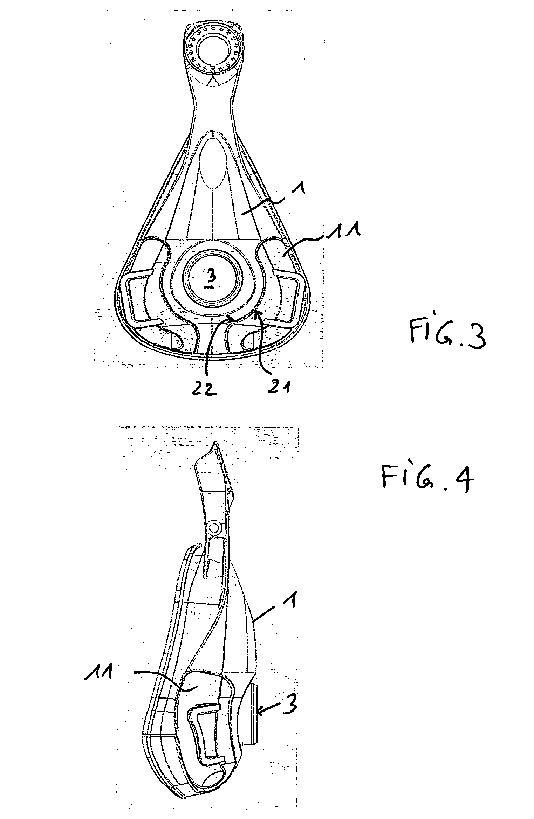

Generally, when two lodgings 12 are provided, one is arranged on each lateral side of the mask body 1 with respect to the central gas inlet or orifice 3, i.e., a right lodging and a left lodging, each of these lodgings 12 receiving a connector element 11 as represented in Figure 3.

The peripheral border 22 or contour of each lodging 12 is conformed and sized so as to have a shape or profile that fits with or matches the general peripheral shape 21, i.e. the contour, of the body 13 of the connector element 11 as visible in Figures 3 to 6.

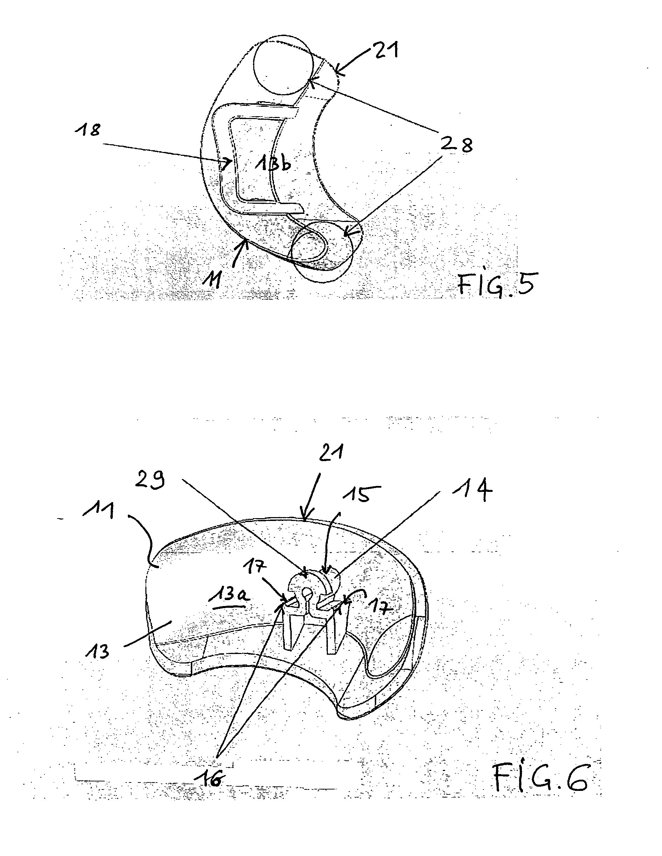

For example, the peripheral shape 21 of the connector element 11 can have a kind of curved shape, such as a"("- or "C"-profile or -shape, or any other suitable profile or shape, as shown in Figures 5 and 6.

The Velcro-type straps 19 of the headgear 9 are linked to each connector element 11 by means of a headgear-connecting structure 18, such as a loop, a buckle or a hook element as shown in Figures 1 and 5. To avoid the patient having to open and close the Velcro-type straps 19 of the headgear 9 for putting the mask into the correct position on his/her face or in contrast, to remove the mask from his/her face, the connector elements 11 and the lodgings 12 comprise a quick coupling/releasing mechanism whose detailed is provided below.

The particular structure of the quick coupling/releasing mechanism for an assembly according to the present invention is shown in Figures 2 and 5 to 12. Actually, the coupling/releasing mechanism comprises elements or means carried by the connector elements 11 and further other elements or means arranged in the lodgings 12. More precisely, each connector element 11 comprises a connector body 13 having an inner face or side 13a and an outer face or side 13b.

As shown in Figures 5 and 6, the outer face 13b bears the headgear-connecting structure 18, such as a loop or a hook element, while the inner face 13a comprises a connecting pin 14 having a semi-cylindrical head 15 and two lateral shoulders 16 (one on each side of the semi-cylindrical head 15). Each shoulder 16 carries a blocking part 17 for preventing or limiting the rotation of the semi-circular or semi-cylindrical head 15 when inserted into the corresponding opening 24 of the internal wall structure 23 of the lodging 12 as explained in more detail below.

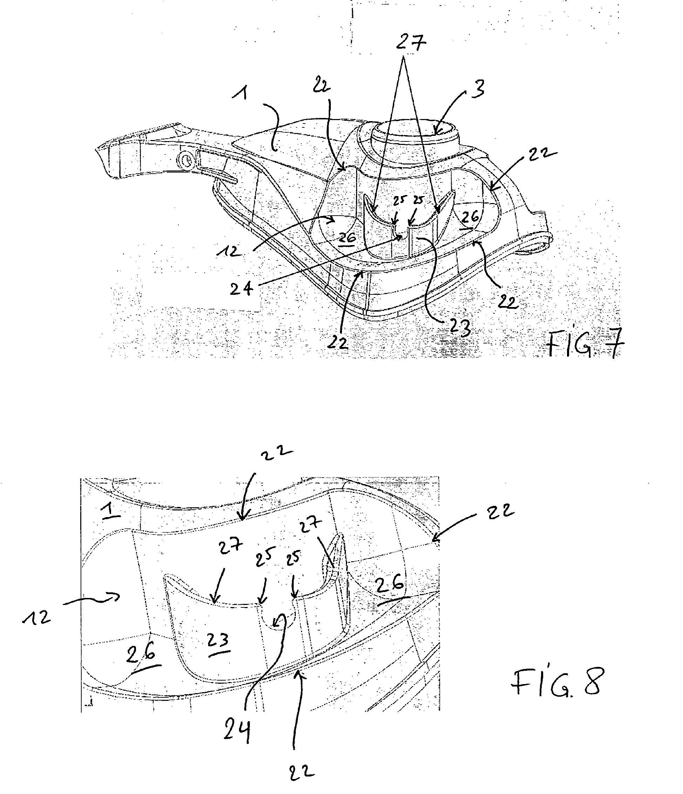

Further, each lodging 12 includes a bottom 26 on which is arranged, i.e. made integral with it, an internal wall structure 23 that projects upward with respect to the bottom 26. The wall structure 23 has an upper border 27 in which an opening 24 is arranged or cut, the opening 24 having a profile that matches the profile of semi-cylindrical head 15 of the connecting pin 14 of the connector element 11, as shown in Figures 7 and 8.

In order to facilitate the insertion of the semi-cylindrical head 15 of the connector element 11 into the opening 24 having a corresponding, i.e. a semi-circular matching shape, and to limit the translation or lateral motion of the semi-cylindrical head 15 in the opening 24, once inserted therein, one or both ends of the semi-cylindrical head 15 of the connector element 11 comprise an abutment 29 as shown in Figure 6. The semi-cylindrical head 15 of the connector element 11 has an elongated semi-cylindrical form.

Actually, in the embodiment shown in Figure 6, only one end of the semi-cylindrical head 15 of the connector element 11 comprises an abutment 29. Indeed, the other end of the semi-cylindrical head 15 of the connector element 11 is in contact with the inner face 13a of the connector body 13, i.e. the internal face 13a of the connector body 13 constitutes the other abutment.

More generally speaking, the connecting pin 14 of the connector element 11 cooperates with the wall structure 23 and the opening 24 to provide a quick and efficient coupling mechanism. Preferably, the entire connecting pin 14 and the connector body 13, and preferably also the hook 18, are moulded in one-piece. Preferably each of the pieces and the resulting moulded one piece are made of a polymer material.

When the semi-cylindrical head 15 of the connecting pin 14 of the connector element 11 is inserted by the user into the semi-circular shaped opening 24 of the wall structure 23 of the lodging 12 of the mask body 1, the semi-cylindrical head 15 penetrates or extends into the opening 24 of the wall structure 23 under the insertion force, i.e. by digital pressure exerted by the user on two actuating zones or areas 28 situated on the outer face 13b of the connector element 11 as shown in Figure 5. Then, the two portions of wall structure 27 forming the two ends 25 delimiting the opening 24 as shown in Figure 8 cooperate with the semi-cylindrical head 15 of the connecting pin 14 of the connector element 11 so as to maintain the connector element 11 locked into the opening 24 of the wall structure 27. Actually, the shape of the opening 24 matches the shape of the semi-cylindrical head 15 so that the wall structure 23 can maintain the semi-cylindrical head 15 in position in the opening 24 formed in the wall structure 23. i.e. the internal border of the opening 24 forms a sort of sleeve or partial sleeve around the semi-cylindrical head 15.

Further, at least one of the lodgings 12, preferably both of them, include a bottom 26 with one or several venting holes 30, preferably several venting holes, therein thereby ensuring a fluid communication between the lodging 12 and the breathing chamber 31. Thus, when the user exhales, expired gases containing carbon dioxide can leave the breathing chamber 31 and be vented through the venting holes 30. Once in the lodgings 12, the vented gases can escape the lodging 12 by passing between the peripheral border 21 of each of the connector elements 11 and the contour 22 of each of the lodgings as a gas tightness is not ensured between the connector elements 11 and the contour 22 of each of the lodgings.

As shown in Figures 9 and 10, at least the bottoms 26 of the lodgings 12 project into the breathing chamber 31 thereby decreasing the dead spaces in the chamber 31.

For coupling the connector element 11 to its corresponding lodging 12, the user has first to position the head 15 of the connecting pin 14 vis-a-vis the opening 24 of the wall structure 27, and then to exert a digital pressure on the pressing areas 28 located on the outer face 13b of the element body 13 as illustrated in Figures 5 and 9. In doing so, the cylindrical-shaped head 15 will pass between the two wall ends 25 of the circular opening 24 and penetrate into the opening 24 of the wall structure 23.

As the wall ends 25 project one toward the other, they will act as abutments that will retain the head 15 into the opening 24, while each shoulder 16 prevents or limits the rotation of the semi-cylindrical head 15 in the opening 24 thanks to the blocking part 17, whereas an abutment 29 prevents the translation slide or motion of the head 15 out of the opening 24.

Actually, a certain force should be exerted by the user for "clipping" the head 15 of the connecting pin 14 into the opening 24 as the head 15 has to pass between the wall ends 25 of the wall structure 23, despite the fact that the width between the wall ends 25 is slightly less than the diameter of the semi-cylindrical head 25. This is possible as the wall structure 23 is made of a material exhibiting a given elasticity, for instance, it is made of a plastic or similar type of material.

For releasing the connector element 11 once it has been inserted and is being held attached to the mask body 1, for example, when the user desires to take off the mask, the user has only to pull on the connector element 11 in order to disengage the head 15 from the opening 24 of the wall structure 23. This disengagement allows for the withdrawal of the connector element 11 which was held in place by the ends wall 25 acting as retaining abutments or nipples.

The assembly of the invention comprises a facial mask can be used in a method for treatment of a respiratory disorder or condition, for example, in non-invasive positive pressure ventilation (NPPV) or in a continuous positive airway pressure (CPAP) therapy of sleep disordered breathing (SDB) conditions, such as, for example, obstructive sleep apnea (OSA).

## Claims

1. Assembly comprising a connector element (11) having a connector body (13), a respiratory mask having a mask body (1), and a coupling/releasing mechanism for coupling or uncoupling said connector element (11) to said mask body (1), the coupling/releasing mechanism comprising a connecting pin (14) comprising a semi-cylindrical head (15), said connecting pin (14) cooperating with a wall structure (23) comprising an upper border (27) in which an opening (24) is arranged, said opening (24) having an internal profile that matches the shape of the semi-cylindrical head (15) of the connecting pin (14), wherein
- the wall structure (23) is situated in a lodging (12) arranged in the external wall of the mask body (1), **characterized in that**:
- the peripheral border (22) of the lodging (12) is conformed and sized to have a profile that matches the peripheral contour (21) of the connector body (13) so that the connector body (13) forms a roof that covers the lodging (12).

2. Assembly according to Claim 1, **characterized in that** the connector element (11) comprises the connecting pin (14), preferably the semi-cylindrical head (15) of the connector element (11) comprises an abutment (29) at one end.

3. Assembly according to any one of the preceding Claims, **characterized in that** the wall structure (23) is a part of the mask body (1).

4. Assembly according to any one of the preceding Claims, **characterized in that** the connector body (13) has an inner face (13a) and an outer face (13b), the inner face (13a) comprising the connecting pin (14) and the outer face (13b) comprising a headgear-connecting structure (18).

5. Assembly according to any one of the preceding Claims, **characterized in that** the connecting pin (14) comprises a semi-cylindrical head (15) and two lateral shoulders (16) with one of the lateral shoulders (16) being arranged on each side of the semi-cylindrical head (15).

6. Assembly according to Claim 5, **characterized in that** each of the two lateral shoulders (16) arranged on each side of the semi-cylindrical head (15) comprises a blocking part (17) for preventing or limiting the rotation of the semi-circular or
semi-cylindrical head (15) while inserted into the opening (24) of the internal wall structure (23).

7. Assembly according to any one of the preceding Claims, **characterized in that** the lodging (12) forms a recess into the external wall of the mask body (1) and further comprises a bottom (26), said mask body (1) comprising a breathing chamber (31) and the bottom (26) of the lodging (12) projecting in the breathing chamber of the mask body (1).

8. Assembly according to any one of the preceding Claims, **characterized in that** the wall structure (23) is carried by the bottom (26) of at least a lodging (12) and projects upwardly with respect to the bottom (26).

9. Assembly according to any one of the preceding Claims, **characterized in that** the wall structure (23) and the mask body are molded into one piece, preferably molded out of a polymer material.

10. Assembly according to any one of the preceding Claims, **characterized in that** the connector body (13), the connecting pin (14) and the headgear-connecting structure (18) are molded into one piece, preferably molded out of a polymer material.

11. Assembly according to any one of the preceding Claims, **characterized in that** the mask body (1) further comprises a gas inlet orifice (3) in fluid communication with the breathing chamber (31), two lodgings (12), each comprising a wall structure (23), being arranged symmetrically with respect to the gas inlet orifice (3).

12. Assembly according to Claim 4, **characterized in that** pressing areas (28) are located on the outer face (13b) of the connector body (13).

13. Assembly according to any one of the preceding Claims, **characterized in that** the peripheral contour (21) of each of the connector body (13) has a curved shape, such as a "(" or "C" shape.

14. Assembly according to any one of the preceding Claims, **characterized in that** the lodging (12) comprises a bottom (26) with at least a venting hole (30),
preferably several venting holes, ensuring a fluid communication between said lodging (12) and the breathing chamber (31).

15. Assembly according to any one of the preceding Claims, **characterized in that** the mask body (1) further comprises a cushion (10) and a headgear (9) with one or more straps (19), at least one of said straps (19) being attached to the headgear-connecting structure (18) of the connector element (11), preferably the mask body (1) further comprises a forehead support (5).

## Patentansprüche

1. Anordnung umfassend ein Anschlusselement (11) mit einem Anschlusskörper (13), eine Atemmaske mit einem Maskenkörper (1) und einen Kopplungs-/Freigabemechanismus zum Koppeln oder Entkoppeln des Anschlusselements (11) an den/von dem Maskenkörper (1), wobei der Kopplungs-/Freigabemechanismus einen Anschlussstift (14) umfasst, der einen halbzylindrischen Kopf (15) umfasst, wobei der Anschlussstift (14) in eine Wandstruktur eingreift (23), welche eine obere Begrenzung (27) umfasst, in der eine Öffnung (24) angeordnet ist, wobei die Öffnung (24) ein Innenprofil hat, das der Form des halbzylindrischen Kopfs (15) des Anschlussstiftes (14) entspricht, wobei
- sich die Wandstruktur (23) in einer Aufnahme (12) befindet, angeordnet innerhalb der Außenwand des Maskenkörpers (1), wobei
- die Abgrenzung (22) der Aufnahme (12) angepasst und bemessen ist, um ein Profil zu haben, das der Außenkontur (21) des Anschlusskörpers (13) entspricht, sodass der Anschlusskörper (13) eine Abdeckung formt, die die Aufnahme (12) abdeckt.

2. Anordnung nach Anspruch 1, **dadurch gekennzeichnet, dass** das Anschlusselement (11) den Anschlussstift (14) umfasst, wobei der halbzylindrische Kopf (15) des Anschlusselements (11) an einem Ende vorzugsweise ein Auflager (29) umfasst.

3. Anordnung nach einem der vorhergehenden Ansprüche, **dadurch gekennzeichnet, dass** die Wandstruktur (23) ein Teil des Maskenkörpers (1) ist.

4. Anordnung nach einem der vorhergehenden Ansprüche, **dadurch gekennzeichnet, dass** der Anschlusskörper (13) eine Innenfläche (13a) und eine Außenfläche (13b) hat, wobei die Innenfläche (13a) den Anschlussstift (14) umfasst, und die Außenfläche (13b) eine Kopfgeschirr-Anschlussstruktur (18) umfasst.

5. Anordnung nach einem der vorhergehenden Ansprüche, **dadurch gekennzeichnet, dass** der Anschlussstift (14) einen halbzylindrischen Kopf (15) und zwei laterale Schultern (16) umfasst, wobei jeweils eine der lateralen Schultern (16) auf jeder Seite des halbzylindrischen Kopfes (15) angeordnet ist.

6. Anordnung nach Anspruch 5, **dadurch gekennzeichnet, dass** jede der beiden lateralen Schultern (16), die auf beiden Seiten des halbzylindrischen Kopfes (15) angeordnet sind, einen Blockierteil (17) zum Verhindern oder zum Beschränken der Rotation des halbrunden oder halbzylindrischen Kopfes (15), während dieser in die Öffnung (24) der inneren Wandstruktur (23) eingeführt ist, umfasst.

7. Anordnung nach einem der vorhergehenden Ansprüche, **dadurch gekennzeichnet, dass** die Aufnahme (12) eine Vertiefung in der Außenwand des Maskenkörpers (1) bildet und ferner einen Boden (26) umfasst, wobei der Maskenkörper (1) eine Beatmungskammer (31) umfasst, und der Boden (26) der Aufnahme (12) in die Beatmungskammer des Maskenkörpers (1) hineinragt.

8. Anordnung nach einem der vorhergehenden Ansprüche, **dadurch gekennzeichnet, dass** die Wandstruktur (23) vom Boden (26) wenigstens einer Aufnahme (12) getragen wird und im Verhältnis zum Boden (26) nach oben ragt.

9. Anordnung nach einem der vorhergehenden Ansprüche, **dadurch gekennzeichnet, dass** die Wandstruktur (23) und der Maskenkörper in einem Teil geformt sind und vorzugsweise aus einem Polymermaterial geformt sind.

10. Anordnung nach einem der vorhergehenden Ansprüche, **dadurch gekennzeichnet, dass** der Anschlusskörper (13), der Anschlussstift (14) und die Kopfgeschirr-Anschlussstruktur (18) in einem Teil geformt sind und vorzugsweise aus einem Polymermaterial geformt sind.

11. Anordnung nach einem der vorhergehenden Ansprüche, **dadurch gekennzeichnet, dass** der Maskenkörper (1) ferner eine Gaseinlassöffnung (3) in Fluidverbindung mit der Beatmungskammer (31) umfasst, wobei zwei Aufnahmen (12), von denen jede eine Wandstruktur (23) umfasst, auf die Gaseinlassöffnung (3) bezogen symmetrisch angeordnet sind.

12. Anordnung nach Anspruch 4, **dadurch gekennzeichnet, dass** Druckbereiche (28) an der Außenoberfläche (13b) des Anschlusskörpers (13) angeordnet sind.

13. Anordnung nach einem der vorhergehenden Ansprüche, **dadurch gekennzeichnet, dass** die Außenkontur (21) von jedem der Anschlusskörper (13) eine gebogene Form, ähnlich "(" oder "C" hat.

14. Anordnung nach einem der vorhergehenden Ansprüche, **dadurch gekennzeichnet, dass** die Aufnahme (12) einen Boden (26) umfasst, der wenigstens eine Lüftungsöffnung (30), vorzugsweise mehrere Lüftungsöffnungen hat, um eine Fluidverbindung zwischen der Aufnahme (12) und der Beatmungskammer (31) sicherzustellen.

15. Anordnung nach einem der vorhergehenden Ansprüche, **dadurch gekennzeichnet, dass** der Maskenkörper (1) ferner ein Polster (10) und ein Kopfgeschirr (9) mit einem oder mehreren Gurten (19) umfasst, wobei wenigstens einer der Gurte (19) mit der Kopfgeschirr-Anschlussstruktur (18) des Anschlusselements (11) verbunden ist, wobei der Maskenkörper (1) vorzugsweise ferner eine Stirnauflage (5) umfasst.

## Revendications

1. Ensemble comprenant un élément connecteur (11) ayant un corps de connecteur (13), un masque respiratoire ayant un corps de masque (1), et un mécanisme de couplage/libération permettant de coupler ou de découpler ledit élément de connecteur (11) dudit corps de masque (1), le mécanisme de couplage/libération comprenant un picot de fixation (14) comprenant une tête semi-cylindrique (15), ledit picot de fixation (14) coopérant avec une structure de paroi (23) comprenant une bordure supérieure (27) dans laquelle une ouverture (24) est agencée, ladite ouverture (24) ayant un profil interne qui concorde avec la forme de la tête semi-cylindrique (15) du picot de fixation (14), dans lequel la structure de paroi (23) est située dans un logement (12) agencé dans la paroi externe du corps de masque (1), **caractérisé en ce que** :
- la bordure périphérique (22) du logement (12) est conformée et dimensionnée pour avoir un profil qui concorde avec le contour périphérique (21) du corps de connecteur (13) de sorte que le corps de connecteur (13) forme un toit qui recouvre le logement (12).

2. Ensemble selon la revendication 1, **caractérisé en ce que** l'élément de connecteur (11) comprend le picot de fixation (14), de préférence la tête semi-cylindrique (15) de l'élément connecteur (11) comprend une butée (29) à une extrémité.

3. Ensemble selon l'une quelconque des revendications précédentes, **caractérisé en ce que** la structure de paroi (23) est une partie du corps de masque (1).

4. Ensemble selon l'une quelconque des revendications précédentes, **caractérisé en ce que** le corps de connecteur (13) a une face interne (13a) et une face externe (13b), la face interne (13a) comprenant le picot de fixation (14) et la face externe (13b) comprenant une structure de fixation de harnais (18).

5. Ensemble selon l'une quelconque des revendications précédentes, **caractérisé en ce que** le picot de fixation (14) comprend une tête semi-cylindrique (15) et deux épaulements latéraux (16), l'un des épaulements latéraux (16) étant agencé de chaque côté de la tête semi-cylindrique (15).

6. Ensemble selon la revendication 5, **caractérisé en ce que** chacun des deux épaulements latéraux (16) agencés de chaque côté de la tête semi-cylindrique (15) comprend une partie de blocage (17) pour empêcher ou limiter la rotation de la tête semi-circulaire ou semi-cylindrique (15) lorsqu'elle est insérée dans l'ouverture (24) de la structure de paroi interne (23).

7. Ensemble selon l'une quelconque des revendications précédentes, **caractérisé en ce que** le logement (12) forme un évidement dans la paroi externe du corps de masque (1) et comprend en outre un fond (26), ledit corps de masque (1) comprenant une chambre de respiration (31) et le fond (26) du logement (12) faisant saillie dans la chambre de respiration du corps de masque (1).

8. Ensemble selon l'une quelconque des revendications précédentes, **caractérisé en ce que** la structure de paroi (23) est portée par le fond (26) d'au moins un logement (12) et fait saillie vers le haut par rapport au fond (26).

9. Ensemble selon l'une quelconque des revendications précédentes, **caractérisé en ce que** la structure de paroi (23) et le corps de masque sont moulés en une seule pièce, de préférence moulés dans un matériau polymère.

10. Ensemble selon l'une quelconque des revendications précédentes, **caractérisé en ce que** le corps de connecteur (13), le picot de fixation (14) et la structure de fixation de harnais (18) sont moulés en une seule pièce, de préférence moulés dans un matériau polymère.

11. Ensemble selon l'une quelconque des revendications précédentes, **caractérisé en ce que** le corps de masque (1) comprend en outre un orifice d'entrée de gaz (3) en communication fluidique avec la chambre de respiration (31), deux logements (12), comprenant chacun une structure de paroi (23), agencés symétriquement par rapport à l'orifice d'entrée de gaz (3).

12. Ensemble selon la revendication 4, **caractérisé en ce que** des zones de pression (28) sont situées sur la face externe (13b) du corps de connecteur (13).

13. Ensemble selon l'une quelconque des revendications précédentes, **caractérisé en ce que** le contour périphérique (21) de chaque corps de connecteur (13) a une forme incurvée, telle qu'une forme de « (» ou de « C ».

14. Ensemble selon l'une quelconque des revendications précédentes, **caractérisé en ce que** le logement (12) comprend un fond (26) doté d'au moins un trou d'aération (30), de préférence plusieurs trous d'aération, garantissant une communication fluidique entre ledit logement (12) et la chambre de respiration (31).

15. Ensemble selon l'une quelconque des revendications précédentes, **caractérisé en ce que** le corps de masque (1) comprend en outre un coussinet (10) et un harnais (9) avec une ou plusieurs sangles (19), au moins l'une desdites sangles (19) étant fixée à la structure de fixation de harnais (18) de l'élément de connecteur (11), de préférence le corps de masque (1) comprend en outre un support frontal (5).
